# EUROPEAN PATENT APPLICATION

(11) **EP 4 414 351 A1**
(43) Date of publication of application: **14.08.2024**
(21) Application number: 22878555.6
(22) Date of filing: 05.10.2022
(51) Int. Cl.: C07C 29/88, C07C 29/80, C07C 31/20

(54) **METHOD FOR PRODUCING HIGH-PURITY 1,3-BUTYLENE GLYCOL**

(30) Priority: 06.10.2021 JP 2021164864
(71) Applicant: KH Neochem Co., Ltd., Tokyo 103-0022 (JP)
(72) Inventor: KAMIHIGASHI, Shun, Yokkaichi-shi, Mie 510-8502 (JP); KUBOKI, Masaharu, Yokkaichi-shi, Mie 510-8502 (JP); KANADA, Jun, Yokkaichi-shi, Mie 510-8502 (JP)
(74) Representative: V.O.
(86) International application number: PCT/JP2022/037298
(87) International publication number: WO 2023/058687

(57) **Abstract**

A production method of high-purity 1,3-butylene glycol including
a step of performing a heat treatment for an initial feed liquid containing 1,3-butylene glycol and
a step of removing low-boiling components that are contained in any one or more of a treated liquid and a treated vapor after the heat treatment by distillation,
in which a content of a base in the initial feed liquid in the step of performing the heat treatment is less than 0.2 mass%.

## Description

### Technical Field

The present invention relates to a production method for high-purity 1,3-butylene glycol that is used in cosmetics or the like.

### Background Art

1,3-Butylene glycol is a viscous, colorless and transparent liquid having a boiling point of 208°C and a low odor and has excellent chemical stability. Therefore, 1,3-butylene glycol is used as a raw material of a variety of synthetic resins and surfactants. 1,3-Butylene glycol is also in use as a material for cosmetics, moisture absorbers, high-boiling point solvents and antifreezes due to its excellent moisture absorption characteristic, low volatility and low toxicity. Particularly, in recent years, the demand for 1,3-butylene glycol has been significantly growing in the cosmetic industry since 1,3-butylene glycol with low toxicity and irritating properties has excellent properties as a moisturizer.

In Patent Literature 1, as a method for improving the yield of odorless 1,3-butylene glycol, a method in which, in performing high-boiling fraction distillation (distillation for removing a high-boiling point component), a high-boiling point component is distilled by adding at least one compound selected from the group consisting of caustic soda, caustic potash, sodium borohydride and potassium borohydride is disclosed.

Patent Literature 2 discloses that an odor substance can be effectively reduced by adding an alkali metal base to 1,3-butylene glycol from which high-boiling components have been removed in advance, performing a heat treatment, then distilling 1,3-butylene glycol away to separate the alkali metal base and the high-boiling components as residues and, subsequently, distilling low-boiling components away from a 1,3-butylene glycol distillate.

### Citation List

### Patent Literature

Patent Literature 1: Japanese Patent Laid-Open No. H7-258129
Patent Literature 2: Japanese Patent No. 4559625

### Summary of Invention

### Technical Problem

However, odorless 1,3-butylene glycol that is obtained by the method described in Patent Literature 1 has a drawback in that a slight odor is generated due to a change over time when stored for a long period of time.

In the vapor of 1,3-butylene glycol that is produced by the method described in Patent Literature 2, odor substances can still be confirmed, and there is a problem with the odors of products. Furthermore, in the production method described in the same literature, an alkali metal base is added to 1,3-butylene glycol from which high-boiling components have been removed in advance, a heat treatment is performed, and then the alkali metal base and the high-boiling components are separated as residues at the same time as the distillation of 1,3-butylene glycol. That is, the alkali metal base is reacted with odor-causing substances to generate high-boiling components, and then the generated high-boiling components are removed together with the alkali metal base from the tower bottom portion of a distillation tower (base removal distillation). In the case of this production method, the alkali metal base is separated by thin-film distillation where the heat history is small and the heating time is short in base removal distillation. However, since the separation capability of thin-film distillation is low, a considerable amount of 1,3-butylene glycol is incorporated into a tower bottom liquid when the generated high-boiling components and the alkali metal base are separated from the tower bottom portion as residues. Therefore, there is a problem in that the yield of a product 1,3-butylene glycol that is obtained in the end decreases.

In consideration of the above-described circumstances, an objective of the present invention is to provide a production method for high-purity 1,3-butylene glycol by which 1,3-butylene glycol from which odor substances in the vapor have been sufficiently reduced can be produced with a favorable yield.

### Solution to Problem

As intensive studies for solving the problems of the above-described production methods, the present inventors found that, when a heat treatment is performed on an initial feed liquid containing 1,3-butylene glycol in a state where the content of a base in the initial feed liquid is set to be less than a specific amount and generated low-boiling components are removed from the tower bottom portion of a distillation tower, since a base removal treatment is unnecessary or the incorporation of 1,3-butylene glycol into the tower bottom liquid in association with the base removal treatment can be suppressed to the minimum extent, it is possible to obtain high-purity 1,3-butylene glycol with a favorable yield and, furthermore, to reduce methyl vinyl ketone, which is one odor component, and a generation source thereof, and thus 1,3-butylene glycol having a low odor can be produced and completed the present invention.

More specifically, the present invention is as described below.
[1] A method for producing high-purity 1,3-butylene glycol comprising:
   a step of performing a heat treatment for an initial feed liquid containing 1,3-butylene glycol; and
   a step of removing low-boiling components that are contained in any one or more of a treated liquid and a treated vapor after the heat treatment by distillation,
   wherein a content of a base in the initial feed liquid in the step of performing the heat treatment is less than 0.2 mass%.
[2] The method for producing high-purity 1,3-butylene glycol according to [1], wherein a heating temperature in the step of performing the heat treatment is 90°C to 200°C, and a heating residence time is 20 minutes to nine hours.
[3] The method for producing high-purity 1,3-butylene glycol according to [1] or [2], wherein the initial feed liquid that is used in the step of performing the heat treatment is generated through a treatment step including a step of removing impurities in 1,3-butylene glycol by extraction and a step of removing a residual extraction solvent after the extraction step by distillation.
[4] High-purity 1,3-butylene glycol, wherein, in GC/MS-olfactometry under the following conditions, a toluene-d8-equivalent concentration of methyl vinyl ketone is 0.25 vol ppb or less.
[5] High-purity 1,3-butylene glycol, wherein, in GC/MS-olfactometry under the following conditions, a toluene-d8-equivalent concentration of methyl vinyl ketone is 0.20 vol ppb or less,
   wherein measurement conditions of the GC/MS-olfactometry are as follows:

### (concentrator)

concentrator: Entech7200 automated concentrator manufactured by Entech Instruments Inc.,
amount of sample: 5 g,
injection amount: 200 mL of a gas-phase part of sample placed still at 30°C for 20 minutes or longer (separately, 100 mL of an inner standard substance apart from the gas-phase part is injected),
inner standard substance: 100 mL (toluene-d8 standard gas: concentration 10 vol ppb, dilution gas is nitrogen, Sumitomo Seika Chemicals Company, Limited.),
concentration method: cold trap dehydration (CTD) mode,
temperature condition of dehydration module 1 (Empty Trap): trap temp. -40°C, desorption temp. 0°C
temperature condition of cold tenax module 2 (Tenax Trap): trap temp. -30°C, desorption temp. 200°C,
temperature condition of focusing module 3 (Cryo focus): trap temp. -165°C, desorption temp. 100°C,
sample flow rate: 50 mL/min,
He flush volume: 75 mL,
M1 to M2 volume: 40 mL (100 mL/min),
M2 to M3 time: 3.0 min.,
injection time: 0.3 min.,

### (GC)

measuring instrument: Agilent 7890B gas chromatography system manufactured by Agilent Technologies, Inc.,
analysis column: DB-1 (column in which a stationary phase is dimethylpolysiloxane; length 60 m × inner diameter 320 µm × film thickness 1 µm) manufactured by Agilent Technologies, Inc.,
temperature rising program: a temperature is retained at 35°C for two minutes, then, raised up to 240°C at 10 °C/minute and retained at 240°C for 7.5 minutes,
carrier gas: helium,
control mode: constant pressure (153.09 kPa),
a concentrated sample is separated with a capillary column and then sent to ODP3 (sniffing system) manufactured by Gestel, Inc. and MSD5977B (mass spectrometer) at a ratio of 1/1,

### (MSD: mass spectrometer)

detector: Agilent 5977B MSD manufactured by Agilent Technologies, Inc.,
ionization mode: EI,
measurement type: scanning,
ion source temperature: 250°C,
quadrupole temperature: 150°C,
electron energy: 70.0 eV,
scanning start mass: 30,
scanning end mass: 400,
calibration curve: produced using a toluene-d8 standard gas (concentration: 10 vol ppb, dilution gas: nitrogen) manufactured by Sumitomo Seika Chemicals Company, Limited,
in data analysis, an EIC peak area (EIC: m/z 55.000) of methyl vinyl ketone that appears at relative retention times of 0.59 to 0.61 when a relative retention time of toluene-d8 is regarded as 1.0 is confirmed using an extracted ion chromatogram (EIC), and, for calculation of the toluene-d8-equivalent concentration, a formula derived from the calibration curve is used.

### Advantageous Effects of Invention

The present invention makes it possible to provide high-purity 1,3-butylene glycol from which odor substances in the vapor have been sufficiently reduced.

The production method for high-purity 1,3-butylene glycol of the present invention makes it possible to achieve reduction of an odor and yield improvement at the same time.

### Brief Description of Drawings

[Figure 1] Figure 1 is an example of a flow sheet of a device for performing a production method of the present invention.
[Figure 2] Figure 2 is an example of a device that is used in GC/MS-olfactometry measurement in the present invention.

### Description of Embodiments

Hereinafter, an embodiment for performing the present invention (hereinafter, referred to as "present embodiment") will be described in detail. The present invention is not limited to the following description and can be carried out after being modified in a variety of manners within the scope of the gist thereof.

In the present embodiment, 1,3-butylene glycol that is the final product will be also referred to as "high-purity 1,3-butylene glycol", and 1,3-butylene glycol as a raw material will be also referred to as "raw material 1,3-butylene glycol" or simply "1,3-butylene glycol".

### [High-Purity 1,3-Butylene Glycol]

In high-purity 1,3-butylene glycol according to the present embodiment, it is preferable that, in GC/MS-olfactometry under specific conditions, a toluene-d8-equivalent concentration of methyl vinyl ketone is 0.25 vol ppb or less.

The measurement conditions of the GC/MS-olfactometry in the present embodiment are as described below.

### [Measurement Conditions of GC/MS-olfactometry]

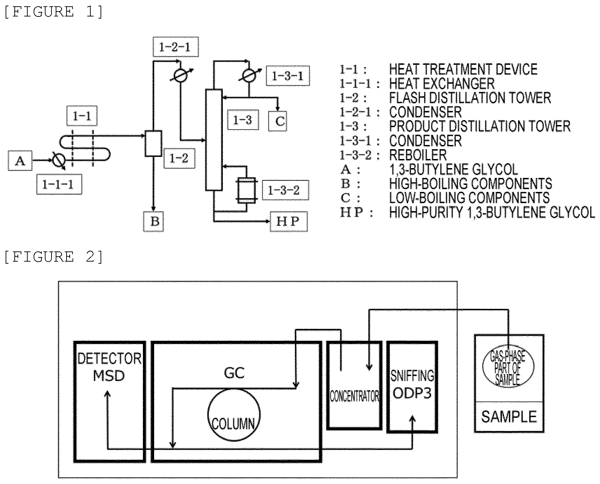

The GC/MS-olfactometry is performed using a device shown in a schematic device view of Figure 2. This device is composed of a concentrator that concentrates the sample vapor of a gas-phase part in a measurement sample, GC that separates the concentrated vapor with a capillary column, a sniffing system that enables the direct smelling of a separated component and a mass spectrometer (MSD) that determines the quality and quantity of the separated component.

### (Concentrator)

Concentrator: Entech7200 automated concentrator manufactured by Entech Instruments Inc.
Amount of sample: 5 g
Injection amount: 200 mL of a gas-phase part of sample placed still at 30°C for 20 minutes or longer (separately, 100 mL of an inner standard substance apart from the gas-phase part is injected).
Inner standard substance: 100 mL (toluene-d8 standard gas: concentration 10 vol ppb, dilution gas is nitrogen, Sumitomo Seika Chemicals Company, Limited.)
Concentration method: Cold trap dehydration (CTD) mode
Temperature condition of dehydration module 1 (Empty Trap): Trap temp. -40°C, desorption temp. 0°C
Temperature condition of cold tenax (R) module 2 (Tenax Trap): Trap temp. -30°C, desorption temp. 200°C,
Temperature condition of focusing module 3 (Cryo focus): trap temp. -165°C, desorption temp. 100°C
Sample flow rate: 50 mL/min
He flush volume: 75 mL
M1 to M2 volume: 40 mL (100 mL/min)
M2 to M3 time: 3.0 min.
Injection time: 0.3 min.

### (GC)

Measuring instrument: Agilent 7890B gas chromatography system manufactured by Agilent Technologies, Inc.
Analysis column: DB-1 (column in which the stationary phase is dimethylpolysiloxane; length 60 m × inner diameter 320 µm × film thickness 1 µm) manufactured by Agilent Technologies, Inc.
Temperature rising program: The temperature is retained at 35°C for two minutes, then raised up to 240°C at 10 °C/minute and retained at 240°C for 7.5 minutes.
Carrier gas: Helium
Control mode: Constant pressure (153.09 kPa)

A concentrated sample is separated with a capillary column and then sent to ODP3 (sniffing system) manufactured by Gestel, Inc. and MSD5977B (mass spectrometer) at a ratio of 1/1.
(MSD: mass spectrometer)
Detector: Agilent 5977B MSD manufactured by Agilent Technologies, Inc.
Ionization mode: EI
Measurement type: Scanning
Ion source temperature: 250°C
Quadrupole temperature: 150°C
Electron energy: 70.0 eV
Scanning start mass: 30
Scanning end mass: 400
Calibration curve: Produced using a toluene-d8 standard gas (concentration: 10 vol ppb, dilution gas: nitrogen) manufactured by Sumitomo Seika Chemicals Company, Limited.

In data analysis, the EIC peak area (EIC: m/z 55.000) of methyl vinyl ketone that appears at relative retention times of 0.59 to 0.61 when the relative retention time of toluene-d8 is regarded as 1.0 is confirmed using an extracted ion chromatogram (EIC). For the calculation of the toluene-d8-equivalent concentration, a formula derived from the calibration curve is used.

When the above-described toluene-d8-equivalent concentration of methyl vinyl ketone is 0.25 vol ppb or less, the odor of high-purity 1,3-butylene glycol tends to be reduced. From the viewpoint of the effect of the present invention becoming more significant, the toluene-d8-equivalent concentration of methyl vinyl ketone is more preferably 0.20 vol ppb or less, still more preferably 0.17 vol ppb or less, far still more preferably 0.16 vol ppb or less and particularly preferably 0.14 vol ppb or less. The lower limit of the volume proportion is not particularly limited and may be, for example, 0.01 vol ppb or more from the viewpoint of the production cost.

In the high-purity 1,3-butylene glycol of the present embodiment, the area percentage of the peak of 1,3-butylene glycol in a gas chromatography analysis under the following conditions is not particularly limited, but is, for example, preferably 99.7% or more, more preferably 99.8% or more and particularly preferably 99.9% or more depending on required product qualities.

### [Conditions of Gas Chromatography Analysis]

Analysis column: DB-WAX (column in which the stationary phase is polyethylene glycol; length 30 m × inner diameter 0.25 mm × film thickness 0.25 µm)
Temperature rising conditions: The temperature is raised from 80°C up to 230°C at 5 °C/minute and then retained at 230°C for 10 minutes.
Sample introduction temperature: 250°C
Carrier gas: Nitrogen
Gas flow rate in column: 0.5 mL/minute
Detector and detection temperature: Flame ionization detector (FID) and 250°C
Control mode: Constant flow
Split ratio: 50:1
Sample injection condition: 1 µL

### [Production Method for High-Purity 1,3-Butylene Glycol]

A production method for high-purity 1,3-butylene glycol of the present embodiment is a method including a step of performing a heat treatment for an initial feed liquid containing 1,3-butylene glycol and a step of removing low-boiling components that are contained in any one or more of a treated liquid and a treated vapor after the heat treatment by distillation.

Hereinafter, the production method for high-purity 1,3-butylene glycol of the present embodiment will be described using Figure 1. Figure 1 is a flow sheet showing an example of a device for performing the production method for high-purity 1,3-butylene glycol of the present embodiment. The device for performing the production method for high-purity 1,3-butylene glycol of the present embodiment is not limited to the aspect shown in Figure 1.

In Figure 1, 1-1 is a heat treatment device, 1-2 is a flash distillation tower, 1-3 is a product distillation tower, 1-1-1 is a heat exchanger, 1-2-1 and 1-3-1 are condensers and 1-3-2 is a reboiler.

In Figure 1, A is 1,3-butylene glycol, B is high-boiling components, C is low-boiling components and HP is high-purity 1,3-butylene glycol.

In the present embodiment, there will be cases where compounds or a mixture having higher boiling points than 1,3-butylene glycol are referred to as "high-boiling components", compounds or a mixture having lower boiling points than 1,3-butylene glycol are referred to as "medium-boiling components" and compounds or a mixture having lower boiling points than the medium-boiling components are referred to as "low-boiling components", respectively.

The high-boiling components are not particularly limited and refer to substances having boiling points of 207°C or higher, and examples thereof include monoester bodies of 1,3-butylene glycol and acetic acid, diester bodies of 1,3-butylene glycol and acetic acid, hydrogenated bodies of a trimer of acetaldehyde, acetal form between acetaldol and 1,3-butylene glycol and the like.

The medium-boiling components are not particularly limited and refer to substances having boiling points of lower than 207°C and 110°C or higher, and examples thereof include 3-hydroxybutanone, 4-hydroxy-2-butanone, acetaldol and the like.

The low-boiling components are not particularly limited and refer to substances having boiling points of lower than 110°C, and examples thereof include acetaldehyde, methyl vinyl ketone, crotonaldehyde and the like.

### [1,3-Butylene Glycol]

In the production method of the present embodiment, 1,3-butylene glycol (A) that is contained in an initial feed liquid as a raw material (hereinafter, also simply referred to as "raw material 1,3-butylene glycol") is not particularly limited, and any of 1,3-butylene glycol produced by the method of liquid phase hydrogen reduction of acetaldol, 1,3-butylene glycol produced by a hydrolysis method of 1,3-butylene oxide, 1,3-butylene glycol produced by a fermentation method in which a microbe or a fungus is used, a mixture thereof and the like may be used. Among these, a reaction product obtained by the method of liquid phase hydrogen reduction of acetaldol is preferably used since the effect of the present invention tends to become more significant. In the method of liquid phase hydrogen reduction of acetaldol, low-boiling components having a unsaturated bond such as acetaldehyde, butyraldehyde, crotonaldehyde, acetone and methyl vinyl ketone, which are considered to be odor-causing substances, or condensation products thereof are generated as byproducts, and it is difficult to completely remove the low-boiling components or the condensation products thereof even by distillation. The odor-causing substance includes a substance that acts as an odor source, and a substance that turns into an odor substance by a change over time, a heat treatment, a chemical treatment or the like.

As the raw material 1,3-butylene glycol, a reaction product obtained by the method of liquid phase hydrogen reduction of acetaldol may also be used after alcohols, salts, moisture and the like are removed. A method for removing the above-described components is not particularly limited, and a method such as distillation or extraction can be used.

In the raw material 1,3-butylene glycol, the area percentage of the peak of 1,3-butylene glycol in the gas chromatography analysis under the above-described conditions is 99.5% or more, more preferably 99.6% or more and still more preferably 99.7% or more since the effect of the invention of present application tends to become more significant.

As 1,3-butylene glycol that is used as the raw material in the production method of the present embodiment, 1,3-butylene glycol from which high-boiling components have been removed is preferably used. When 1,3-butylene glycol containing a small amount of high-boiling components is used as the raw material, low-boiling components that are generated by a decomposition reaction of high-boiling components in the heat treatment step are not generated or generated in an extremely small quantity, and thus it becomes possible to efficiently remove the low-boiling components, which act as odor causes, in the subsequent distillation step. As a result, it is conceivable that the absolute amount of the low-boiling components can be reduced to near zero with no limitations, and thus it becomes possible to produce 1,3-butylene glycol having an extremely high quality in which an odor is suppressed and a change over time is small.

1,3-Butylene glycol from which a high-boiling components have been removed is not particularly limited, and examples thereof include 1,3-butylene glycol from which impurities have been removed by extraction and a residual extraction solvent has been removed by distillation (hereinafter, referred to as "extraction treatment"), 1,3-butylene glycol after high-boiling fraction distillation, 1,3-butylene glycol obtained from a product distillation tower in a conventional purification process, a low-quality product 1,3-butylene glycol, a product 1,3-butylene glycol from an unknown origin, a mixture thereof or the like and preferably include 1,3-butylene glycol after the extraction treatment.

The content of high-boiling components in 1,3-butylene glycol from which high-boiling components have been removed in the present embodiment is preferably 1.0 mass% or less, more preferably 0.5 mass% or less and still more preferably 0.3 mass% or less. When the content of high-boiling components in 1,3-butylene glycol is 1.0 mass% or less, the generation of odor-causing substances due to the decomposition of high-boiling components in the heat treatment step tends to be further suppressed.

### [Heat Treatment Step]

The production method for high-purity 1,3-butylene glycol of the present embodiment includes a step of performing a heat treatment for an initial feed liquid containing 1,3-butylene glycol. When a heat treatment is performed on the initial feed liquid containing 1,3-butylene glycol, medium-boiling components and/or high-boiling components that are contained in the initial feed liquid are decomposed to turn into low-boiling components, and thus it is presumed that the low-boiling components that become odor-causing in the subsequent distillation step are efficiently removed. However, the mechanism of the present invention is not limited to what has been described above.

In the heat treatment step, the initial feed liquid containing 1,3-butylene glycol, which serves as the raw material, is supplied to the heat treatment device 1-1. The raw material 1,3-butylene glycol that is charged into the heat treatment device 1-1 may be any 1,3-butylene glycol; however, particularly, 1,3-butylene glycol from which high-boiling components have been removed is preferable. For example, the raw material 1,3-butylene glycol may be 1,3-butylene glycol distilled from a high-boiling component distillation tower or the product 1,3-butylene glycol that is obtained from the tower bottom portion of a product distillation tower in a conventional method. Examples of the conventional method include product distillation described in Patent Literature 2, but the present invention is not limited to what has been described above.

The initial feed liquid that is used in the step of performing the heat treatment is preferably purified by, particularly, a treatment step including a step of removing impurities in 1,3-butylene glycol by extraction and a step of removing a residual extraction solvent after the extraction step by distillation since the effect of the present invention tends to become significant.

The heating residence time in the heat treatment step is not particularly limited, but is preferably 20 minutes to nine hours, more preferably one to six hours and still more preferably one to two hours. When the heating residence time is 20 minutes or longer, the turning of medium-boiling components and/or high-boiling components into low-boiling components tends to progress more sufficiently, and when the heating residence time is nine hours or shorter, an increase in the cost taken for the heat treatment tends to be further suppressible.

The heating temperature in the heat treatment step is not particularly limited, but is preferably 90°C to 200°C, more preferably 120°C to 190°C and still more preferably 120°C to 140°C. When the heating temperature is 90°C or higher, the turning of medium-boiling components and/or high-boiling components into low-boiling components tends to progress more sufficiently, and, when the heating temperature is 200°C or lower, the generation of odor-causing substances due to the thermal decomposition of 1,3-butylene glycol tends to be further suppressed.

A heat treatment device in the heat treatment step is not particularly limited, examples thereof include heat treatment devices such as a continuous tube-type device, a batch tank-type device and a continuous tank-type device, and, among these, the batch tank-type device is preferable from the viewpoint of odor substance reduction.

In the heat treatment step, a base may be contained in the initial feed liquid that is charged into the heat treatment device. In this case, the content of the base in the initial feed liquid is less than 0.2 mass%, preferably 0.05 mass% or less and more preferably 0.03 mass% or less. In a case where the content of the base in the initial feed liquid is less than 0.2 mass%, since a base removal treatment is unnecessary or the incorporation of 1,3-butylene glycol into the tower bottom liquid in association with the base removal treatment can be further suppressed to the minimum extent, it becomes possible to improve the yield of high-purity 1,3-butylene glycol.

The base that is contained in the initial feed liquid is not particularly limited, and examples thereof include alkali metal compounds such as caustic soda (NaOH), caustic potash (KOH), sodium carbonate (Na₂CO₃), sodium bicarbonate (NaHCOs), potassium carbonate (K₂CO₃) and potassium bicarbonate (KHCO₃).

### [High-boiling component Removal Step]

The production method for high-purity 1,3-butylene glycol of the present embodiment may have a high-boiling component removal step before a low-boiling component removal step to be described below. In the high-boiling component removal step, any one or more of a treated liquid and a treated vapor that have been subjected to the heat treatment in the heat treatment step are supplied to the flash distillation tower 1-2, and high-boiling components (B) are removed from the distillation tower bottom portion. An operation of returning and circulating the removed high-boiling components (B) in the flash distillation tower 1-2 may be performed.

In a case where a base is contained in the initial feed liquid in the heat treatment step, the base may be removed together with the high-boiling components (B) by flash distillation or may be separately supplied to a base removal tower (not shown) and removed. An evaporator that is used in the base removal tower is not particularly limited, but a gravity flow-type thin film evaporator or forced stirring-type thin film evaporator where the heating time is short is preferably used.

In the flash distillation tower 1-2, evaporation is performed under reduced pressure of 13.3 kPa or lower, preferably 0.7 to 2.7 kPa, in the tower top portion. From the viewpoint of further suppressing the odor of 1,3-butylene glycol, the distillation temperature is preferably 120°C to 190°C, more preferably 140°C to 170°C and particularly preferably 150°C to 170°C. The residence time is preferably 20 minutes to nine hours, more preferably one to six hours and still more preferably one to two hours. 1,3-Butylene glycol containing low-boiling components is distilled from the tower top portion of the flash distillation tower 1-2 and sent to the subsequent product distillation tower 1-3.

In the high-boiling component removal step, other distillation means such as an evaporator and a distillation tower may be used in addition to the flash distillation.

### [Low-boiling component Removal Step]

Any one or more of the treated liquid and the treated vapor discharged from the heat treatment device 1-1 is subjected to the high-boiling component removal step as necessary, then, sent to the product distillation tower 1-3 and subjected to a low-boiling component removal step. In the product distillation tower 1-3, high-purity 1,3-butylene glycol (HP) is extracted from the tower bottom portion, and low-boiling components (C) are distilled away from the tower top portion.

The product distillation tower 1-3 is not particularly limited, and a perforated plate tower, a bubble cap tower, a packed tower or the like can be used; however, a packed tower being packed with a regular packing or an irregular packing and having a low pressure loss is preferably more appropriate. This is to lower the distillation temperature as much as possible since 1,3-butylene glycol is thermally decomposed at a temperature of higher than 200°C and adversely affects the odor. In addition, even in a case where a heat history (heating time) that is applied to 1,3-butylene glycol is long, the influence is the same. Therefore, a reboiler that is adopted may be, for example, a vertical thermosiphon type, a horizontal thermosiphon type, an overflow tube bundle type (kettle type), a forced circulation type or an insertion type; however, for example, a thin film evaporator such as a gravity flow-type thin film evaporator or forced stirring-type thin film evaporator where the heating time of a process-side fluid is short is preferably appropriate.

The product distillation tower 1-3 is preferably a packed tower having seven to 40 theoretical plates. The product distillation tower 1-3 may be one tower or two or more towers may be used. As the distillation conditions, the pressure at the tower top portion of the product distillation tower 1-3 is preferably 1 to 20 kPa, and the temperature at the tower bottom portion of the product distillation tower 1-3 is preferably 100°C to 160°C and more preferably 110°C to 140°C. The product distillation tower 1-3 is preferably operated at a reflux ratio of 0 to 5.0. Examples of a specific aspect of the low-boiling point substance removal step include a method in which a liquid or vapor containing a large amount of 1,3-butylene glycol is continuously supplied to the distillation tower, a distillate containing a large amount of the low-boiling point component is continuously extracted from the tower top portion and, simultaneously, high-purity 1,3-butylene glycol is continuously extracted from the tower bottom portion.

In the description of Figure 1, a tower top portion vapor of the flash distillation tower 1-2 is concentrated with the condenser 1-2-1, and a concentrated liquid is supplied to the product distillation tower 1-3; however, from the viewpoint of reducing the amount of heat for heating in the product distillation tower 1-3, the treated liquid from the heat treatment device 1-1 may be directly supplied to the product distillation tower 1-3. High-purity 1,3-butylene glycol (HP) can be obtained from the tower bottom portion of the product distillation tower 1-3.

In the present embodiment, it is also possible to obtain high-purity 1,3-butylene glycol (HP) from the tower top portion or in the middle of the concentration stage by, first, distilling the low-boiling components away from the treated liquid discharged from the heat treatment device 1-1 with the product distillation tower 1-3 and, subsequently, distilling or evaporating 1,3-butylene glycol extracted in the middle of the concentration stage or from the tower bottom portion to separate the base and the generated high-boiling components as distillation residues.

In addition, high-purity 1,3-butylene glycol (HP) obtained by distilling the low-boiling components (C) away from the product distillation tower 1-3 may be further distilled away to remove the high-boiling components, a part of high-purity 1,3-butylene glycol (HP) from which the low-boiling components (C) have been distilled away may be recycled to the flash distillation tower 1-2 or the low-boiling components (C) containing 1,3-butylene glycol may be recycled to the heat treatment device 1-1.

In the present embodiment, it becomes possible to produce high-purity 1,3-butylene glycol with a favorable yield by performing at least the step of performing the heat treatment for the initial feed liquid having a base content of less than 0.2 mass% and containing 1,3-butylene glycol and the step of removing the low-boiling components that are contained in any one or more of the treated liquid and the treated vapor after the heat treatment by distillation. Here, the range of "high purity" changes depending on the use of high-purity 1,3-butylene glycol and high purity means that 1,3-butylene glycol has a purity high enough to be used in a specific use. For example, in a case where 1,3-butylene glycol is used in a cosmetic use, the area percentage of the peak of high-purity 1,3-butylene glycol in the gas chromatography analysis under the above-described conditions is preferably 99.70% to 100%, more preferably 99.75% to 100% and still more preferably 99.78% to 100%.

### Examples

Hereinafter, the present embodiment will be specifically described with examples and comparative examples, but the present invention is not limited thereto. In the following examples and comparative examples, a device that is shown in a flow sheet of Figure 1 was used.

Regarding high-purity 1,3-butylene glycols obtained in the examples and the comparative examples, a variety of physical properties were measured according to the following.

### 1. GC/MS-olfactometry

Methyl vinyl ketone that was contained in each of 1,3-butylene glycols obtained in Examples 1 to 8 and Comparative Example 1 was measured by a GC/MS-olfactometry analysis according to the following method. The GC/MS-olfactometry was performed using a device shown in a schematic device view of Figure 2. This device is composed of a concentrator that concentrates the sample vapor of a gas-phase part in a measurement sample, GC that separates the concentrated vapor with a capillary column, a sniffing system that enables the direct smelling of a separated component and a mass spectrometer (MSD) that determines the quality and quantity of the separated component.

### (Concentrator)

Concentrator: Entech7200 automated concentrator manufactured by Entech Instruments Inc.
Amount of sample: 5 g
Injection amount: 200 mL of a gas-phase part of sample placed still at 30°C for 20 minutes or longer (separately, 100 mL of an inner standard substance apart from the gas-phase part was injected)
Inner standard substance: 100 mL (toluene-d8 standard gas: concentration 10 vol ppb, dilution gas was nitrogen, Sumitomo Seika Chemicals Company, Limited.)
Concentration method: Cold trap dehydration (CTD) mode
Temperature condition of dehydration module 1 (Empty Trap): Trap temp. -40°C, desorption temp. 0°C
Temperature condition of cold tenax (R) module 2 (Tenax Trap): Trap temp. -30°C, desorption temp. 200°C
Temperature condition of focusing module 3 (Cryo focus): Trap temp. -165°C, desorption temp. 100°C
Sample flow rate: 50 mL/min
He flush volume: 75 mL
M1 to M2 volume: 40 mL (100 mL/min)
M2 to M3 time: 3.0 min.
Injection time: 0.3 min.

### (GC)

Measuring instrument: Agilent 7890B gas chromatography system manufactured by Agilent Technologies, Inc.
Analysis column: DB-1 (column in which the stationary phase is dimethylpolysiloxane; length 60 m × inner diameter 320 µm × film thickness 1 µm) manufactured by Agilent Technologies, Inc.
Temperature rising program: The temperature was retained at 35°C for two minutes, then, raised up to 240°C at 10 °C/minute and retained at 240°C for 7.5 minutes
Carrier gas: Helium
Control mode: Constant pressure (153.09 kPa)

A concentrated sample is separated with a capillary column and then sent to ODP3 (sniffing system) manufactured by Gestel, Inc. and MSD5977B (mass spectrometer) at a ratio of 1/1
(MSD: mass spectrometer)
Detector: Agilent 5977B MSD manufactured by Agilent Technologies, Inc.
Ionization mode: EI
Measurement type: scanning
Ion source temperature: 250°C
Quadrupole temperature: 150°C
Electron energy: 70.0 eV
Scanning start mass: 30
Scanning end mass: 400
Calibration curve: Produced using a toluene-d8 standard gas (concentration: 10 vol ppb, dilution gas: nitrogen) manufactured by Sumitomo Seika Chemicals Company, Limited

In data analysis, the EIC peak area (EIC: m/z 55.000) of methyl vinyl ketone that appeared at relative retention times of 0.59 to 0.61 when the relative retention time of toluene-d8 was regarded as 1.0 was confirmed using an extracted ion chromatogram (EIC). The toluene-d8-equivalent concentration of methyl vinyl ketone in the vapor of 1,3-butylene glycol was calculated using "toluene-d8-equivalent concentration (vol ppm) = 3.15 × 10⁻⁶ × EIC peak area", which was a formula derived from the calibration curve.

### 2. GC Measurement

GC measurement was performed on 1,3-butylene glycol in a distillate obtained from the tower top portion for flash distillation in each of Example 2, Example 3 and Comparative Example 1 and 1,3-butylene glycol as a raw material according to the following method, and the area percentage (%) of each peak component was obtained.
Analysis device: Agilent Technologies 7890BGCSystem manufactured by Agilent Technologies, Inc.
Detector: FID (flame ionization detector)
Analysis column: DB-WAX (column in which the stationary phase is polyethylene glycol; 30 m × 250 µm × 0.25 µm) manufactured by Agilent Technologies, Inc.
Temperature rising program: the temperature was raised from 80°C at 5 °C/minute and retained at 230°C for 10 minutes
INJ: 250°C
DET: 250°C
Control mode: constant flow
Split ratio: 50:1
Sample injection condition: 1 µL

It was considered that peaks that appeared at retention times of nine to 15 minutes was from low-boiling components, peaks that appeared at retention times of 15 to 17 minutes was from medium-boiling components, peaks that appeared at retention times of 17 to 19 minutes was from 1,3-butylene glycol and peaks that appeared at retention times of 19 to 25 minutes was from high-boiling components, and the area percentage (%) of the peaks for each was measured.

### [Example 1]

As 1,3-butylene glycol (A) as a raw material, and 1,3-butylene glycol-P (trade name) manufactured by KH Neochem Co., Ltd. were used.

100 parts by mass of 1,3-butylene glycol (A) was charged into a continuous tube-type heat treatment device 1-1. At this time, a 10 mass% caustic soda aqueous solution was added so that the caustic soda concentration reached 0.03 mass% relative to an initial feed liquid. The temperature in the heat treatment device 1-1 was maintained at 120°C, and a heat treatment was performed for 20 minutes.

Next, a treated liquid discharged from the heat treatment device 1-1 was supplied to a flash distillation tower 1-2. In the flash distillation tower 1-2, the temperature was set to 130°C, the pressure was set to 1.1 kPa, and 2 parts by mass of caustic soda, high-boiling components (B) and a part of 1,3-butylene glycol relative to 100 parts by mass of the amount of the initial feed liquid were discharged from the tower bottom portion. 98 parts by mass of 1,3-butylene glycol and low-boiling components were distilled from the tower top portion of the flash distillation tower 1-2 and supplied to a subsequent product distillation tower 1-3. In the product distillation tower 1-3, a distillation device equipped with a 500 mm-high packed tower (inner diameter: 22 mm) packed with 3 mm-sized Dixon rings such that the packed height reached 425 mm was used, the temperature was set to 110°C, the pressure was set to 1.1 kPa, 10 parts by mass of low-boiling components (C) and a part of 1,3-butylene glycol relative to 100 parts by mass of the amount of the initial feed liquid were distilled from the tower top portion, and high-purity 1,3-butylene glycol (HP) was brought out from the tower bottom portion.

In the obtained high-purity 1,3-butylene glycol (HP), the toluene-d8-equivalent concentration of methyl vinyl ketone, which is an odor-causing substance, measured by sniffing GC/MS was 0.24 vol ppb. The results are shown in Table 1.

### [Example 2]

The process was performed in the same manner as in Example 1 except that the caustic soda concentration in the continuous tube-type heat treatment device 1-1 was set to 0 mass%. In the obtained high-purity 1,3-butylene glycol (HP), the toluene-d8-equivalent concentration of methyl vinyl ketone, which is an odor-causing substance, was 0.20 vol ppb. The results are shown in Table 1.

### [Example 3]

The process was performed in the same manner as in Example 2 except that the heating time in the continuous tube-type heat treatment device 1-1 was set to 60 minutes. In the obtained high-purity 1,3-butylene glycol (HP), the toluene-d8-equivalent concentration of methyl vinyl ketone, which is an odor-causing substance, was 0.17 vol ppb. The results are shown in Table 1.

### [Example 4]

The process was performed in the same manner as in Example 3 except that distillation in the flash distillation tower 1-2 was omitted and the treated liquid discharged from the continuous tube-type heat treatment device 1-1 was directly supplied to the product distillation tower 1-3. In the obtained high-purity 1,3-butylene glycol (HP), the toluene-d8-equivalent concentration of methyl vinyl ketone, which is an odor-causing substance, was 0.16 vol ppb. The results are shown in Table 1.

### [Example 5]

The process was performed in the same manner as in Example 4 except that, instead of the continuous tube-type heat treatment device 1-1, a batch tank-type heat treatment device was used, the temperature in the heat treatment device was maintained at 120°C, and the heat treatment was performed for 60 minutes. In the obtained high-purity 1,3-butylene glycol (HP), the toluene-d8-equivalent concentration of methyl vinyl ketone, which is an odor-causing substance, was 0.14 vol ppb. The results are shown in Table 1.

### [Example 6]

The process was performed in the same manner as in Example 5 except that the temperature in the batch tank-type heat treatment device was maintained at 160°C, the heat treatment was performed for 360 minutes, and the temperature and pressure during product distillation were set to 130°C and 2.9 kPa. In the obtained high-purity 1,3-butylene glycol (HP), the toluene-d8-equivalent concentration of methyl vinyl ketone, which is an odor-causing substance, was 0.14 vol ppb. The results are shown in Table 1.

### [Example 7]

The process was performed in the same manner as in Example 6 except that the temperature in the batch tank-type heat treatment device was maintained at 190°C and the heat treatment was performed for 120 minutes. In the obtained high-purity 1,3-butylene glycol (HP), the toluene-d8-equivalent concentration of methyl vinyl ketone, which is an odor-causing substance, was 0.11 vol ppb. The results are shown in Table 1.

### [Example 8]

The process was performed in the same manner as in Example 5 except that, in the product distillation tower 1-3, 3 parts of the low-boiling components (C) and a part of 1,3-butylene glycol relative to 100 parts of the amount of the initial feed liquid were distilled from the tower top portion. In the obtained high-purity 1,3-butylene glycol (HP), the toluene-d8-equivalent concentration of methyl vinyl ketone, which is an odor-causing substance, was 0.10 vol ppb. The results are shown in Table 1.

### [Comparative Example 1]

The process was performed in the same manner as in Example 1 except that the caustic soda concentration in the heat treatment device was set to 0.2 mass%. In the obtained high-purity 1,3-butylene glycol (HP), the toluene-d8-equivalent concentration of methyl vinyl ketone, which is an odor-causing substance, was 0.38 vol ppb. The results are shown in Table 1.

**[Table 1]**

| | Methyl vinyl ketone concentration (toluene-d8-equivalent concentration) | Heat treatment device | NaOH concentration | Heating residence time | Heating temperature | Flash distillation | Yield |
|---|---|---|---|---|---|---|---|
| | Vol ppb | | Mass% | Minutes | °C | | % |
| Comparative Example 1 | 0.38 | Continuous tube type | 0.2 | 20 | 120 | Performed | 88.9 |
| Example 1 | 0.24 | Continuous tube type | 0.03 | 20 | 120 | Performed | 88.3 |
| Example 2 | 0.20 | Continuous tube type | 0 | 20 | 120 | Performed | 88.2 |
| Example 3 | 0.17 | Continuous tube type | 0 | 60 | 120 | Performed | 88.6 |
| Example 4 | 0.16 | Continuous tube type | 0 | 60 | 120 | Omitted | 90.8 |
| Example 5 | 0.14 | Batch tank type | 0 | 60 | 120 | Omitted | 90.4 |
| Example 6 | 0.14 | Batch tank type | 0 | 360 | 160 | Omitted | 90.0 |
| Example 7 | 0.11 | Batch tank type | 0 | 120 | 190 | Omitted | 90.0 |
| Example 8 | 0.10 | Batch tank type | 0 | 60 | 120 | Omitted | 97.0 |

### [GC Measurement]

GC measurement was performed on 1,3-butylene glycol in a distillate obtained from the tower top portion for flash distillation in each of Example 2, Example 3 and Comparative Example 1 and 1,3-butylene glycol as a raw material, and the area percentage (%) of each peak component was obtained. The results are shown in Table 2. In the table, 1,3-BG represents 1,3-butylene glycol.

**[Table 2]**

| | | | | | |
|---|---|---|---|---|---|
| Retention time (minute) | 9-15 | 15-17 | 17-19 | 19-25 | 9-25 |

| Peak component | Low-boiling components | Medium-boiling components | 1,3-BG | High-boiling components | All components |
|---|---|---|---|---|---|
| | Area percentage | Area percentage | Area percentage | Area percentage | Area percentage |
| Raw material 1,3-butylene glycol (A) | 0.001 | 0.009 | 99.812 | 0.178 | 100.000 |
| Comparative Example 1 flash distillation distillate | 0.000 | 0.008 | 99.812 | 0.180 | 100.000 |
| Example 2 flash distillation distillate | 0.002 | 0.005 | 99.819 | 0.174 | 100.000 |
| Example 3 flash distillation distillate | 0.004 | 0.004 | 99.815 | 0.177 | 100.000 |

It is suggested that, in the heat treatment step of Comparative Example 1, the low-boiling components decreased and the high-boiling components increased. On the other hand, it is suggested that, in the heat treatment steps of Example 2 and Example 3, the medium-boiling components and the high-boiling components decreased and the low-boiling components increased.

### Industrial Applicability

High-purity 1,3-butylene glycol that is provided by the production method of the present invention is industrially available as a raw material of synthetic resins, a raw material of surfactants, a solvent, an antifreeze, a cosmetic raw material or the like.

## Claims

1. A method for producing high-purity 1,3-butylene glycol comprising:
a step of performing a heat treatment for an initial feed liquid containing 1,3-butylene glycol; and
a step of removing low-boiling components that are contained in any one or more of a treated liquid and a treated vapor after the heat treatment by distillation,
wherein a content of a base in the initial feed liquid in the step of performing the heat treatment is less than 0.2 mass%.

2. The method for producing high-purity 1,3-butylene glycol according to claim 1, wherein a heating temperature in the step of performing the heat treatment is 90°C to 200°C, and a heating residence time is 20 minutes to nine hours.

3. The method for producing high-purity 1,3-butylene glycol according to claim 1 or 2, wherein the initial feed liquid that is used in the step of performing the heat treatment is generated through a treatment step including a step of removing impurities in 1,3-butylene glycol by extraction and a step of removing a residual extraction solvent after the extraction step by distillation.

4. High-purity 1,3-butylene glycol, wherein in GC/MS-olfactometry under the following conditions, a toluene-d8-equivalent concentration of methyl vinyl ketone is 0.25 vol ppb or less.

5. High-purity 1,3-butylene glycol, wherein in GC/MS-olfactometry under the following conditions, a toluene-d8-equivalent concentration of methyl vinyl ketone is 0.20 vol ppb or less,
wherein measurement conditions of the GC/MS-olfactometry are as follows:
(concentrator)
concentrator: Entech7200 automated concentrator manufactured by Entech Instruments Inc.,
amount of sample: 5 g,
injection amount: 200 mL of a gas-phase part of sample placed still at 30°C for 20 minutes or longer (separately, 100 mL of an inner standard substance apart from the gas-phase part is injected),
inner standard substance: 100 mL (toluene-d8 standard gas: concentration 10 vol ppb, dilution gas is nitrogen, Sumitomo Seika Chemicals Company, Limited.),
concentration method: cold trap dehydration (CTD) mode,
temperature condition of dehydration module 1 (Empty Trap): trap temp. -40°C, desorption temp. 0°C
temperature condition of cold tenax module 2 (Tenax Trap): trap temp. -30°C, desorption temp. 200°C,
temperature condition of focusing module 3 (Cryo focus): trap temp. -165°C, desorption temp. 100°C,
sample flow rate: 50 mL/min,
He flush volume: 75 mL,
M1 to M2 volume: 40 mL (100 mL/min),
M2 to M3 time: 3.0 min.,
injection time: 0.3 min.,
(GC)
measuring instrument: Agilent 7890B gas chromatography system manufactured by Agilent Technologies, Inc.,
analysis column: DB-1 (column in which a stationary phase is dimethylpolysiloxane; length 60 m × inner diameter 320 µm × film thickness 1 µm) manufactured by Agilent Technologies, Inc.,
temperature rising program: a temperature is retained at 35°C for two minutes, then, raised up to 240°C at 10 °C/minute and retained at 240°C for 7.5 minutes,
carrier gas: helium,
control mode: constant pressure (153.09 kPa),
a concentrated sample is separated with a capillary column and then sent to ODP3 (sniffing system) manufactured by Gestel, Inc. and MSD5977B (mass spectrometer) at a ratio of 1/1,
(MSD: mass spectrometer)
detector: Agilent 5977B MSD manufactured by Agilent Technologies, Inc.,
ionization mode: EI,
measurement type: scanning,
ion source temperature: 250°C,
quadrupole temperature: 150°C,
electron energy: 70.0 eV,
scanning start mass: 30,
scanning end mass: 400,
calibration curve: produced using a toluene-d8 standard gas (concentration: 10 vol ppb, dilution gas: nitrogen) manufactured by Sumitomo Seika Chemicals Company, Limited,
in data analysis, an EIC peak area (EIC: m/z 55.000) of methyl vinyl ketone that appears at relative retention times of 0.59 to 0.61 when a relative retention time of toluene-d8 is regarded as 1.0 is confirmed using an extracted ion chromatogram (EIC), and, for calculation of the toluene-d8-equivalent concentration, a formula derived from the calibration curve is used.
